# EUROPEAN PATENT SPECIFICATION

(11) **EP 0 709 680 B1**
(45) Date of publication and mention of the grant of the patent: **26.09.2001**
(21) Application number: 94919875.8
(22) Date of filing: 08.07.1994
(51) Int. Cl.: G01N 33/553, G01N 33/548

(54) **MAGNETIC PARTICLES COVERED WITH GELATIN AND IMMUNOASSAY USING THE SAME**
MAGNETISCHE MIT GELATINE ÜBERZOGENE TEILCHEN UND SIE VERWENDENDER IMMUNOASSAY
PARTICULES MAGNETIQUES COUVERTES DE GELATINE ET IMMUNO-ESSAI LES UTILISANT

(30) Priority: 08.07.1993 JP 19318493
(43) Date of publication of application: 01.05.1996
(73) Proprietor: FUJIREBIO Inc., Shinjuku-ku, Tokyo 163-07 (JP)
(72) Inventor: ISOMURA, Mitsuo, Shinjuku-ku Tokyo 163-07 (JP); UENO, Masayoshi, Shinjuku-ku Tokyo 163-07 (JP); SHIMADA, Kazuya, Shinjuku-ku Tokyo 163-07 (JP); ASHIHARA, Yoshihiro, Shinjuku-ku Tokyo 163-07 (JP)
(74) Representative: Gillard, Marie-Louise
(86) International application number: JP9401120
(87) International publication number: WO9502185

(56) References cited:
- WO-A-92/08134
- JP-A- 3 115 862
- JP-A- 52 082 723
- JP-A- 59 195 161
- JP-T- 3 504 303

## Description

### FIELD OF THE INVENTION

This invention relates to magnetic particles and an immunoassay method using the same. More particularly, it relates to an immunoassay method which uses magnetic particles having a particle size of 1.0 to 10 *µ* m and comprising an organic polymer material as a core, a magnetic material layer coating said core and gelatin coating said outer layer.

### BACKGROUND OF THE INVENTION

In immunoassay, in particular enzyme immunoassays, beads having a fairly large diameter have so far been used as the solid phase. It is known that a reduction in their size and an increase in solid phase surface area are advantageously conductive to high levels of sensitivity in carrying out immunological reactions.

However, when particles having a smaller diameter are used, a centrifuge must be used or filtration using a filter must be made for B/F separation, so that the assay method can hardly be said to be simple and easy. Therefore, for carrying out B/F separation efficiently and simply, it has been proposed that magnetic particles small in diameter are employed.

Thus, for instance, an immunoassay method is known in which particles produced by coating a magnetite core with a silane and having a particle size of 1.0 to 10 *µ* m was used (Japanese Kokai Tokkyo Koho Sho-55-141670 and Sho-50-122997).

Another immunoassay method was known in which particles produced by coating a magnetic metal oxide core with a silane and having a particle size of 0.1 to 1.5 *µ* m was used (Japanese Kokai Tokkyo Koho Sho-60-1564).

However, these known particles comprising a magnetic metal material as a core have some drawbacks. For instance, they are poorly uniform in particle size, or have poor long-storage stability as exemplified by iron migration.

Accordingly, attempts have been made to improve the particles and stabilize them for long-period storage. Thus, for instance, particles comprising organic polymer materials as the core and a surface coating layer composed of an iron oxide-based ferrite and having a particle size of 0.2 to 3 *µ* m and an immunoassay method using the same are known (Japanese Kokai Tokkyo Koho Hei-3-115862) corresponding to EP-A-420 186. Further, gelatin-ferrite particles produced by dispersing ferromagnetic fine particles in gelatin and having a particle size of 1.0 to 100 *µ* m and an immunoassay method using the same are known (Japanese Kokoku Tokkyo Koho Hei-3-17103).

Furthermore, an immune complex transfer assay method has been proposed for the purpose of attaining noise reduction (Tanpakushitsu, Kakusan, Koso, vol. 37, pp. 144).

WO 92/08134 discloses colloidal particles having a solid core coated with at least one water-soluble gelatine.

### DISCLOSURE OF THE INVENTION

The magnetic particles comprising organic polymer materials as a core and an iron oxide-based ferrite surface layer have indeed improved in long-storage stability but some problems have still been unsolved. For instance, they are inferior in floatability at the time of immune reaction and show a tendency toward particle aggregation at the time of B/F separation in the process of immunoassay and, furthermore, have poor redispersibility. As regards the gelatin ferrite particles produced by dispersing ferromagnetic fine particles in gelatin, the problem of particle aggregation has been solved but, however, their magnetic response property is poor, hence they are not suited for immunoassay, particularly, in enzyme immunoassay, in which rapid B/F separation and washing are required. The immune complex transfer assay method is not practical since it includes an increased number of procedural steps and required a fairly long assay time.

This inventors made earnest efforts to solve these problems and found that magnetic particles comprising organic polymer materials as a core, a magnetic material layer formed to coat the core and a gelatin layer coating the surface of the magnetic material layer and having a particle size of 1.0 to 10 *µ* m, when used in immunoassay, can result in low-noise and high-signal in the assay, owing to the fact that the magnetic particles are stable over a long period of time, thus can be stored for a long period, have been improved in the floatability, furthermore, hardly tend to undergo self-aggregation and show sufficient magnetic response and dispersibility to enable rapid B/F separation and washing. Furthermore, the magnetic particles of this invention can be produced with ease and in a manner such that they have a desired particle size and are uniform in particle size as well. This fact, too, may be said to result in a high level of reproducibility.

This invention thus provides an immunoassay method using gelatin-coated magnetic particles comprising a core consisting of organic polymer materials, a magnetic material layer coating the surface of said core, and a gelatin layer coating the surface of said magnetic material layer and having a particle size of 1.0 to 10 *µ* m.

As the magnetic particles that can be used in the method of this invention, there may be mentioned, for example, those produced by using magnetic particles comprising a layer formed from a magnetic material on the surface of a core made of organic polymer materials and coating said magnetic particles with gelatin. (Hereinafter such particles are referred to as gelatin-coated magnetic particles.)

The above-mentioned method coating with gelatin can be carried out by any method in general use in the art, for example by the chemical binding method utilizing the functional groups on the magnetic particle surface and of gelatin or by the technique of coacervation [T. N. Evreinova(author): "Coacervates", Modern Biology Series 22, Kyoritsu Shuppan].

The chemical binding method utilizing the functional groups on the magnetic particle surface and of gelatin can be carried out, for example, by subjecting the carboxyl, amino, hydroxyl and/or thiol groups on the magnetic particle surface and the amino or carboxyl groups of gelatin to be condensed by dehydration.

For the dehydration, any condensing agent generally used in peptide bond formation reactions can be used. Examples are water-soluble carbodiimides and Woodward's reagents. Coupling thiol groups on the magnetic particle surface to gelatin, the method comprising introducing in advance maleimide groups into gelatin and submitting the maleimidated gelatin to the reaction can be used, among others.

As coating gelatin by coacervation, the method comprising coating the outer surface of magnetic particles with gelatin particles prepared by coacervation, using the chemical binding method, and the method which comprises carrying out coacervation of gelatin in the presence of magnetic particles, are applicable.

The term "coacervation" as used herein means coacervate formation, and "coacervate" means the whole system composed of a solute-rich phase and a solute-poor phase.

While two types of coacervation, simple and complex, are distinguishable depending on the conditions, either type may be used in producing the particles of this invention.

In simple coacervation, the phenomenon was used that the solubility of the organic polymer decreases and phase separation occurs of said organic polymer from the aqueous solution, for instance, when an electrolyte or an organic solvent is added to a solution of an organic polymer or when the pH of a solution of an organic polymer becomes equal to the isoelectric point of the organic polymer. In complex coacervation, an electric interaction resulting from the combination of a polycation and a polyanion causes coacervation. In this case, the mixing ratio between the polycation and polyanion, the initial concentration, the coexisting salt and the concentration thereof, and the pH are determining factors.

In a process for producing the gelatin-coated magnetic particles as given by way of example, a solution containing gelatin (2 to 10%), a metaphosphate salt (0.01 to 5%), magnetic particles (0.3 to 1%) and a water-soluble polysaccharide (0 to 5%) are used and an acid is added to thereby adjust the pH to 2.5 to 6.0, whereupon coacervation occurs to give gelatin-coated magnetic particles. Then, the gelatin layer is reacted with an aldehyde crosslinking agent for insolubilization of said outer layer. Gelatin used for coating magnetic particles is a kind of derived protein and is obtained from collagen. Among various species, acid-treated gelatin having an isoelectric point of 6 to 9 is desirable. As the water-soluble polysaccharide, for example, gum arabic, carboxymethylcellulose, sodium alginate, agar and carrageenan are usable.

The particle size measurement of particles in the dispersion medium can be performed using a particle size measuring apparatus employing the laser diffraction technique, for example an LA-500 apparatus (Horiba Seisakusho). The gelatin-coated magnetic particles of this invention have a mean particle diameter of 1 to 10 *µ* m. For more efficient immunoassay, however, the mean particle size is preferably within the range of 2 to 8 *µ* m. Smaller particles in particle size than 1 *µ* m require a long time for B/F separation and render it difficult to perform the assay in a simple and easy manner. Particles with a particle size exceeding 10 *µ* m are disadvantageous in that the floatability becomes inferior, hence assays cannot be performed with high sensitivity.

For producing gelatin-coated magnetic particles having a particle size of 1 to 10 *µ* m, the particle size of core is preferably within the range of 0.1 to 5 *µ* m. When a core having a particle size larger than 5 *µ* m are used, a particle size of the produced gelatin-coated magnetic particles will be larger than 10 *µ* m. When a core having a particle size smaller than 0.1 *µ* m are used, the gelatin-coated magnetic particles will have a particle size smaller than 1 *µ* m, rendering it impossible to conduct assays efficiently. The organic polymer materials to be used as the core material for the gelatin-coated magnetic particles includes polystyrene, polymethacrylic esters, polyacrylic esters, styrene-methacrylic ester copolymers, styrene-acrylic ester copolymers, polymerized silanes and organic silanes, among others.

The magnetic material to form a core-coating layer is a magnetic metal material and use may be made of ferrite, manganese-, cobalt-, nickel- or like metal-containing ferrite species and the like.

When an antibody or antigen is bound thereto, the gelatin-coated magnetic particles of this invention can be used in immunoassay. The antibody to be bound to the particles includes, among others, antibodies to drug substances such as theophylline, phenytoin and valproic acid, to low-molecular-weight hormones such as thyroxine, estrogen and estradiol, to cancer markers such as CEA and AFP, to viral antigens such as HIV, ATLA and HBV, to macromolecular hormones such as TSH and insulin, to cytokines such as IL-1, IL-2 and IL-6, to various growth factors such as EGF and PDGF, and, further, to appropriate DNAs, RNAs and the like of the viruses mentioned above. The antigen to be used includes viruses such as HIV, ATLA and HBV, DNAs of such viruses and macromolecular hormones such as insulin and TSH, among others.

For binding an antigen or antibody to the gelatin-coated magnetic particles, the physical adsorption method or the chemical binding method can be used. The physical adsorption is carried out by reacting said particles with an antigen or antibody in an appropriate buffer solution. The buffer to be used in this method is, for example, phosphate buffer, Trishydrochloride buffer, carbonate buffer or the like. When both materials are mixed at room temperature, the reaction readily progresses to give the desired product. In the case of chemical binding, the carbodiimide method so called among peptide bond formation methods can be employed. Thus, for instance, for effecting the reaction, a water-soluble carbodiimide solution is added to an equal amount of a 0.1 to 5% dispersion of gelatin-coated magnetic particles under acidic conditions (pH 4 to 6), the reaction is carried out at room temperature for 10 minutes to 1 hour, the supernatant is then removed, and an antibody or antigen solution having a concentration of 0.01 to 10.0 mg/ml, preferably 0.1 to 5 mg/ml, is added for binding of the antibody or antigen. As the buffer to be used on that occasion, phosphate buffer and the like are preferred. It is also possible to effect the chemical binding according to other methods in the presence of a bivalent crosslinking agent such as glutaric anhydride, N-succinimidyl-4-maleimidoburytic acid (GMBS), N-succinimidyl-4-(N-maleimidomethyl)cyclohexanecarboxylic acid (CHMS), glutaraldehyde or cyanuric chloride [cf. "Peputido Goseiho (Peptide Synthesis)", Maruzen Co. (1975); "Koso Men-eki Sokuteiho (Enzyme immunoassay)", Kyoritsu Shuppan; "Tanpakushitsu, kakusan, Koso (Protein, Nucleic acid, Enzyme)", Supplement No. 31 (1987)].

The immunoassay method of this invention can be performed in the manner of radioimmunoassay or enzyme immunoassay, among others. These assay methods use a labeled antigen or antibody and the target antigen or antibody can be measured by the sandwich or competition technique.

In the enzyme immunoassay method, antibody-bound gelatin-coated magnetic particles are reacted with an enzyme-labeled antibody and a sample for 1 minute to 18 hours. The reaction temperature is within the range of 4°C to 40°C, preferably 25°C to 38°C. The unreacted enzyme-labeled antibody is washed away, and that, an appropriate substrate is added and the activity of the antibody-bound enzyme bound to the solid phase is determined, whereby the ligand in the sample can be quantitated.

The enzyme to be used for labeling includes, among others, peroxidase, alkaline phosphatase, β-galactosidase and glucose oxidase. The substrate to be used in the assay should, of course, be one adapted to the enzyme employed and, thus, includes, among others, ABTS, luminol-H₂O₂ (for peroxidase), p-nitrophenyl phosphate, methylumbelliferyl phosphate, 3-(2'-spiroadamantane)-4-methoxy-4-(3"-phosphoryloxy)phenyl-1,2-dioxetane (hereinafter referred to as AMPPD) (for alkaline phosphatase), p-nitrophenyl- β -0-galactose, methylumbelliferyl-β -O-galactose (for β-galactosidase), and the like.

The measurement of the quantitation is performed by allowing the reaction to proceed at 4°C to 40°C for 1 minute to 18 hours and then measuring the resulting color development, fluorescence intensity or luminescence intensity. For the measurement, the so-called rate method to be conducted with warming in the range of 4°C to 40°C may also be employed.

The radioimmunoassay uses a radioisotope such as ¹²⁵I as the label in lieu of the enzyme label mentioned above. The procedure is generally the same as in the enzyme immunoassay mentioned above, except that radioactivity is measured.

Radiolabeling of antigens or antibodies can be readily performed using the Bolton-Hunter reagent already available on the market. Thus, for example, radiolabeling can be conducted with ease by adding said Bolton-Hunter reagent to an antigen or antibody solution in 0.1 M aqueous sodium hydrogen carbonate and, after the lapse of 1 to 2 hours, by removing the unreacted Bolton-Hunter reagent using a G-25 desalting column, for instance.

The chloramine T method or Iodogen method, for instance, can also be employed for effecting ¹²⁵I labeling with ease.

For allowing the immune reaction to proceed, a sample is added to the gelatin-coated magnetic particles of this invention, and the mixture is maintained at 4°C to 40°C, preferably 20°C to 38°C, for 1 minute to 18 hours. Then the particles are washed with physiological saline or distilled water, a raiolabeled antibody is added to the gelatin-coated magnetic particles, and the reaction is allowed to proceed at 4°C to 40°C, preferably 20°C to 38°C, for 1 minute to 18 hours, followed by washing with physiological saline or distilled water, and radioactivity is measured using a scintillation counter.

The assay method of this invention can also be performed in the manner of chemiluminescence assay or immunofluorescence assay. In the former, isoluminol, an acridine ester or the like is used as the label and, in the latter, fluorescein, rhodamine or the like is used as the label. In these cases, labeling can easily be made using the activated ester method or isocyanate method ("Koso Men-eki Sokuteiho (Enzyme immunoassay)", Igaku Shoin,1987).

Similarly, in an example of antibody assaying, antigenbound gelatin-coated magnetic particles according to this invention are mixed with a sample and the reaction is allowed to proceed at 4°C to 40°C for 1 minute to 18 hours, the particles are then washed with physiological saline or distilled water, a labeled anti-human immunoglobulin antibody or a labeled antigen is further added, the reaction is allowed to proceed at 4°C to 40°C for 1 minute to 18 hours, washing is conducted and the activity of the label is measured.

As mentioned above, the immunoassay method according to the invention is the one that magnetic particles comprising an organic polymer core, a magnetic material layer coating the core and gelatin applied on the surface of the magnetic material layer are used. This invension can be used as an immunoassay method capable of giving low-noise, high-signal data.

### BRIEF DESCRIPTION OF THE DRAWINGS

Fig. 1 shows comparative supernatant turbidity data obtained by placing magnetic particles, gelatin ferrite particles and gelatin-coated magnetic particles, in a dispersed form, in respective cells of a spectrophotometer and allowing the dispersions to stand.

Fig. 2 shows comparative supernatant turbidity data obtained by subjecting magnetic particles, gelatin ferrite particles and gelatin-coated magnetic particles, in a dispersed form, to magnetic separation.

Fig. 3 comparatively shows degrees of aggregation of alkaline phosphatase-bound magnetic particles and alkaline phosphatase-bound gelatin-coated magnetic particles as found after magnetic separation, standing, and addition of a substrate.

Fig. 4 comparatively shows degrees of aggregation of alkaline phosphatase-bound magnetic particles and alkaline phosphatase-bound gelatin-coated magnetic particles as found after magnetic separation, standing, and addition of a substrate.

Fig. 5 comparatively shows signal values obtained with magnetic particles and gelatin-coated magnetic particles.

Fig. 6 comparatively shows blank values obtained with magnetic particles and gelatin-coated magnetic particles.

Fig. 7 comparatively shows S/N ratios obtained with magnetic particles and gelatin-coated magnetic particles.

Fig. 8 comparatively shows signal values obtained with magnetic particles and gelatin-coated magnetic particles.

Fig. 9 comparatively shows blank values obtained with magnetic particles and gelatin-coated magnetic particles.

Fig. 10 comparatively shows S/N ratios obtained with magnetic particles and gelatin-coated magnetic particles.

### BEST MODES FOR CARRYING OUT THE INVENTION

The following examples are further illustrative of this invention.

### Example 1

### [Production of macromolecule particles]

A polymerization vessel equipped with a stirrer, thermometer, monomer dropping funnel, reflux condenser, heater and nitrogen gas inlet was charged with 230 parts of deionized water. Then, at 80°C, 1 part of a monomer mixture (A) composed of styrene, 2-ethylhexyl acrylate and ethylene glycol dimethacrylate in the proportions of 80/10/10 and 10 parts of a 10% aqueous ammonium persulfate solution were added and then 99 parts of the same monomer mixture (A) as mentioned above was added dropwise over 3 hours, whereupon macromolecule particles were obtained. Electron microscopy revealed that these particles were practically in a monodisperse state and had a particle size of 0.3 *µ* m.

### Example 2

### [Production of magnetic particles]

A magnetic material formation apparatus equipped with a stirrer, thermometer, oxidizing agent solution, dropping funnel, heater and nitrogen gas inlet tube was charged with 100 parts of the organic polymer materials particle emulsion obtained as mentioned above (solid content: 30%). Nitrogen gas was introduced thereinto for freeing the core emulsion from oxygen.

Then, 100 parts of a ferrous chloride solution (solid content: 40 parts) and 150 parts of ammonium acetate (solid content: 75 parts), each prepared in advance were charged into the apparatus and the contents were heated to 70°C with thorough stirring. Thereafter, the pH was adjusted to 7.2 with aqueous ammonia while stirring was continued.

One hundred fifty parts of a sodium nitrite solution (solid content: 15 parts) was added by dropwise to the resultant over about 1 hour. During the dropping, the liquid phase was maintained, with continued nitrogen gas introduction and stirring, at a temperature of 70°C and a pH within the range of 7.0 to 7.2 to form a ferrite coating on the surface of said particles. After about 20 minutes, the solution was cooled and filtered, and the solid was repeatedly washed with deionized water. Ferrite magnetic particles were thus recovered.

### Example 3

Acid-treated gelatin (0.4 g) with an isoelectric point of pH 9 was dissolved in 20 ml of warm water at 40°C, and the solution was adjusted to pH 9 with sodium hydroxide. This mixed solution was poured into 15 ml of 30% (by volume) ethyl alcohol solution warmed in advance to 40°C , followed by thorough stirring. To this were added 80 *µ* l of 10% sodium hexametaphosphate solution, 0.1 ml of 10% alkyl sulfomaleate and 0.2 g of the magnetic particles produced in Example 2, followed by thorough stirring. Then, the mixture was adjusted to pH 5.0 by dropwise addition of 10% (by volume) acetic acid solution while the mixture was maintained at 40°C . Glutaraldehyde (65 mg) was added to the thus-formed particle dispersion while the latter was maintained at 4°C . The resultant mixture was stirred and then allowed to stand at 4 °C overnight. This was washed with 0.9% NaCl and then made up into a 10% particle dispersion with 0.9% NaCl. An equal volume of 4% (by volume) formaldehyde solution was added and, after dispersion, the mixture was allowed to stand at 4°C for 1 week. Measurement with an LA-500 apparatus revealed that the thus-obtained gelatin-coated magnetic particles had a mean diameter of 6.2 *µ* m.

### Example 4

Acid-treated gelatin (0.4 g) with an isoelectric point of pH 9 was dissolved in 10 ml of warm water at 40°C, and the solution was adjusted to pH 9 with sodium hydroxide. To this solution was added 0.4 g of gum arabic dissolved in 10 ml of water as warmed to 40°C , followed by thorough stirring. This mixed solution was poured into 15 ml of 30% (by volume) ethyl alcohol solution warmed in advance to 40°C , followed by thorough stirring. To this were added 80 *µ* l of 10% sodium hexametaphosphate solution, 0.1 ml of 10% alkyl sulfomaleate and 0.2 g of the magnetic particles produced in Example 2, followed by thorough stirring. Then, the mixture was adjusted to pH 5.0 by dropwise addition of 10% (by volume) acetic acid solution while the mixture was maintained at 40°C . Glutaraldehyde (65 mg) was added to the thus-formed particle dispersion while the latter was maintained at 4°C . The resultant mixture was stirred well and then allowed to stand at 4°C overnight. This was washed with 0.9% NaCl and then made up into a 10% particle dispersion with 0.9% NaCl. An equal volume of 4% (by volume) formaldehyde solution was added and, after effecting dispersion, the mixture was allowed to stand at 4°C for 1 week. Measurement with an LA-500 apparatus revealed that the thus-obtained gelatin-coated magnetic particles had a mean diameter of 4.7 *µ* m.

### Example 5

### [Investigation on particle floatability]

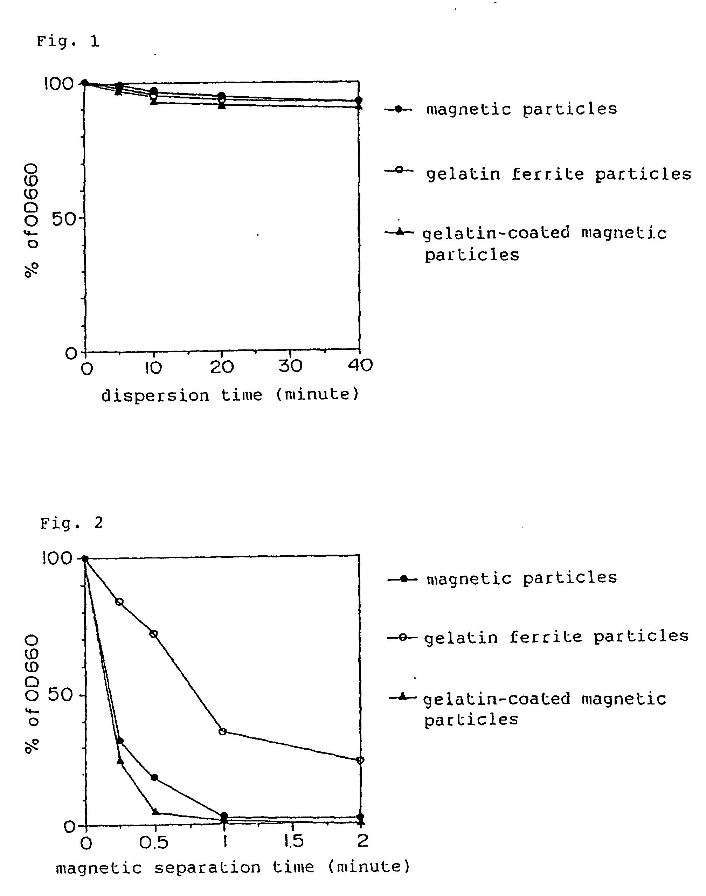

The gelatin-coated magnetic particles produced in Example 3, the magnetic particles produced in Example 2 (for comparison) and gelatin-ferrite particles with ferromagnetic particles were dispersed in gelatin (Japanese Kokoku Tokkyo Koho Hei-3-17103) (for comparison) were respectively dispersed, in a concentration of 0.015%, in 100 mM Tris-hydrochloride-150 mM NaCl buffer (pH 7.2) containing 2% BSA (bovine serum albumin). One ml of each dispersion was placed in a cell for a spectrophotometer (Hitachi) and allowed to stand at room temperature. After 5 to 40 minutes, the turbidity of the supernatant was determined by measuring the absorbance at wavelength 660 nm. The relative turbidity data thus obtained are shown in Fig. 1.

### Example 6

### [Comparison of particle species with respect to magnetic separability]

The gelatin-coated magnetic particles produced in Example 3, the magnetic particles produced in Example 2 (for comparison) and gelatin-ferrite particles with ferromagnetic particles were dispersed in gelatin (Japanese Kokoku Tokkyo Koho Hei-3-17103) (for comparison) were respectively dispersed, in a concentration of 0.015%, in 100 mM Tris-hydrochloride-150 mM NaCl buffer (pH 7.2) containing 2% BSA. One ml of each dispersion was placed in a tube and the tube was brought into contact with a magnet with a surface magnetic field strength of 1000 gauss. After 15 seconds to 2 minutes of contacting with a magnet, the turbidity of the supernatant was determined by measuring the absorbance at wavelength 660 nm. The relative turbidity data thus obtained are shown in Fig. 2.

### Example 7

### [Comparison of particle species with respect to aggregation tendency under drying conditions]

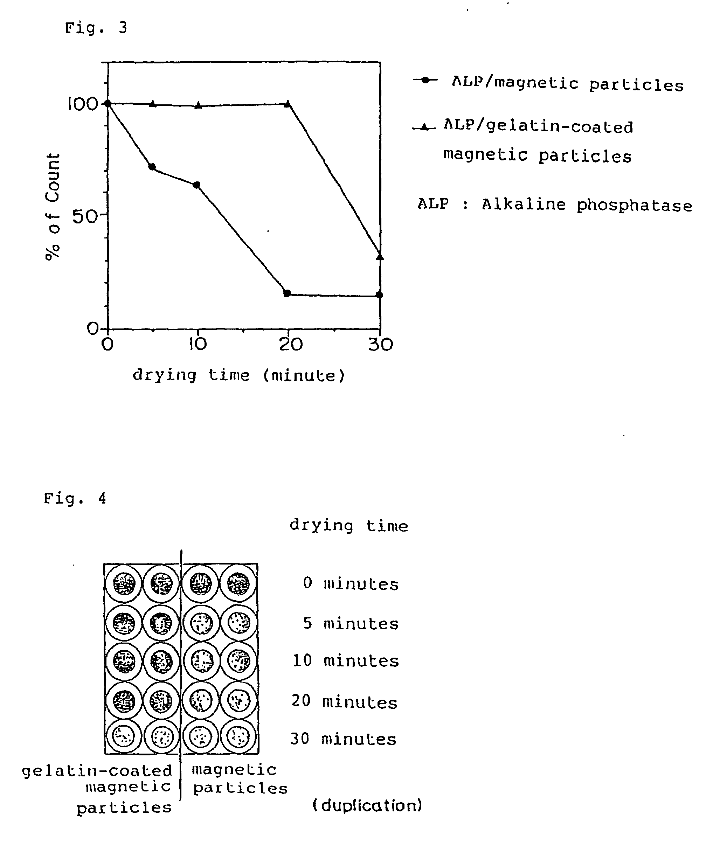

A 250-*µ* 1 portion of a dispersion (0.015%) of the magnetic particles produced in Example 2 or the gelatin-coated magnetic particles produced in Example 3 as bound with alkaline phosphatase was placed in a cartridge and subjected to magnetic separation by contacting the cartridge with a magnet, and the supernatant was removed. Further, washing was performed by adding 300 *µ* l of 0.9% NaCl, followed by magnetic separation. Washing was further repeated two times with 0.9% NaCl and three times with distilled water. The particles separated as magnetically were allowed to stand at room temperature for 0 to 30 minutes. Then, the luminescent substrate, AMPPD, was added and the reaction was allowed to proceed at 37°C for 5 minutes. Immediately thereafter, the luminescence intensity was measured using a luminometer (ALOKA). The relations thus obtained between particles drying (standing) time and signal value are shown in Fig. 3. A photograph of dispersion of said particles as supplemented with the luminescent substrate and placed on an ELISA plate is shown in Fig. 4.

### Example 8

### [Carboxylation of particles]

A 100-mg portion of the gelatin-coated magnetic particles produced in Example 3 were washed three times each with distilled water, water and distilled water, then 50 mg of glutaric anhydride suspended to 5% by volume in 0.1M sodium hydrogen carbonate was added thereto, and the reaction was allowed to proceed for 10 minutes. Thereafter, the particles were washed three times with 0.1 M sodium hydrogen carbonate solution and five times with distilled water. The particles thus obtained are referred to as carboxylated particles.

### Example 9

### [Preparation of anti-AFP antibody bound carboxylated particles]

A 25-mg portion of the carboxylated particles produced in Example 8 were dispersed in 2.5 ml of 10 mM phosphate buffer (pH 4.5), and 25 mg of a water-soluble carbodiimide was added to the dispersion. After allowing the reaction to proceed at room temperature for 20 minutes, the supernatant was removed, 2.5 ml of an anti-AFP antibody solution (1 mg/ml, 20 mM phosphate buffer, pH 4.5) was added, and the mixture was stirred with an end-over-end mixer. After 2 hours, the particles were washed five times with a 2% BSA solution (0.1 M Tris-hydrochloride, 1 mM MgCl₂, pH 7.5) and then dispersed in the same BSA solution.

### Example 10

### [AFP assay using anti-AFP antibody bound carboxylated particles]

In a cartridge, 10 *µ* l of a sample containing 200 ng/ml of AFP antigen was mixed with 250 *µ* l of the dispersion of the carboxylated particles bound to anti-AFP antibody prepared in Example 9, and the reaction was allowed to proceed at 37°C for 10 minutes. The cartridge was contacted with a magnet with a surface magnetic field with strength of 3000 gauss to thereby cause magnetic separation of the particles. The supernatant was removed by decantation. Three hundred *µ* l of physiological saline was added to the particles and, after stirring and magnetic separation, the supernatant was discarded by decantation. This procedure was repeated three times. Then, the particles were mixed with 250 *µ* l of the solution of an alkaline phosphatase bound anti-AFP antibodies (Fab') (0.1 *µ* g/ml, 0.1 M Trishydrochloride, 1 mM MgCl₂, 0.1 mM ZnCl₂), and the reaction was allowed to proceed at 37°C for 10 minutes. The particles were washed by decantation using physiological saline. A substrate solution containing 200 *µ* g/ml of the luminescent substrate, AMPPD (0.1 M DEA-hydrochloride, 1 mM MgCl₂, pH 10.0), was added to the cartridge containing the particles treated as above, and the reaction was allowed to proceed at 37°C for 5 minutes. Thereafter, the luminescence intensity was measured using a luminometer (ALOKA).

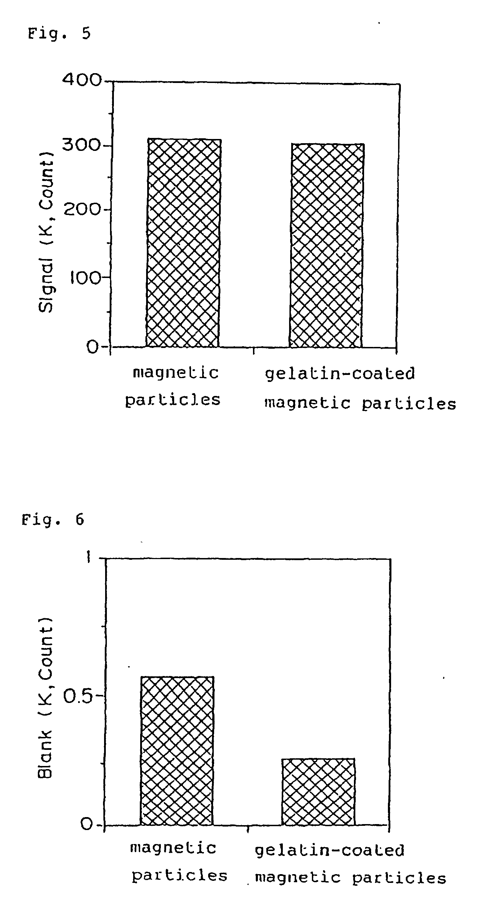

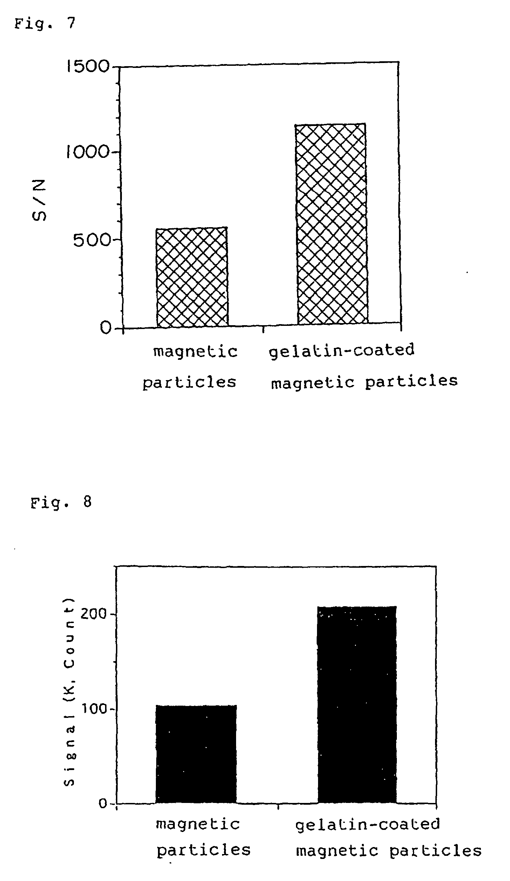

Separately, the magnetic particles produced in Example 2 was aminosilanated, then carboxylated, and then, anti-AFP antibodies were bound to the particles, which were then used for assaying AFP. The obtained results are shown in Figs. 5, 6 and 7. Signal values are given in Fig. 5. blank values (noise values) in Fig. 6, and S/N ratios in Fig. 7.

### Example 11

### [Maleimidation of gelatin-coated magnetic particles]

A 20-mg portion of the gelatin-coated magnetic particles prepared in Example 3 was dispersed in 4 ml of 100 mM sodium phosphate buffer (pH 7.0), and 2 ml of a GMBS solution (1 mg/ml, ethanol) was added, and the mixture was stirred in an end-over-end manner. After an hour, 2 ml of the same GMBS solution was added and the mixture was stirred in an end-over-end manner. After an hour, the particles were washed three times with ethanol and further three times with 100 mM sodium phosphate buffer (pH 7.0).

### Example 12

### [Preparation of TP antigen bound particles]

3.5 *µ* g of iminothiolane was added to 350 *µ* l of antigen solution (300 *µ* g/ml, 50 mM aqueous sodium hydrogen carbonate, pH 8.2) of a Treponema pallidum (TP) in a solution form. After allowing the reaction to proceed at 37 °C for 30 minutes, the buffer was replaced with 50 mM potassium phosphate buffer (1 mM EDTA·2Na, pH 7.0) using PD-10 (Pharmacia). In this mixture, 3.5 mg of the maleimidated particles prepared in Example 11 were dispersed. After 2 hours of stirring using an end-over-end mixer, the particles were washed three times with 50 mM sodium phosphate buffer (1 mM EDTA·2Na, pH 7.0) and further three times with a 2% BSA solution (50 mM Tris-hydrochloride, 0.15 M sodium chloride, 1 mM EDTA·2Na, pH 7.2) and then were dispersed in the same 2% BSA solution to give a TP antigen bound particle dispersion.

### Example 13

### [Antibody detection assay using TP antigen bound particles]

In a cartridge, 20 *µ* l of a 10-fold dilution of positive serum for anti-TP antibody was mixed with 350 *µ* l of the 0.01% dispersion of the particles bound TP antigens which was prepared in Example 12, and the reaction was allowed to proceed at 37°C for 10 minutes. The cartridge was contacted with a magnet with a surface magnetic field with strength of 3000 gauss for magnetic separation of the particles, and the supernatant was discarded by decantation. Then, 300 *µ* l of physiological saline was added, and the mixture was stirred.

The cartridge was drained in the same manner as mentioned above. This procedure was repeated three times. Then, 250 *µ* l of the solution of an alkaline phosphatase bound anti-human antibody (0.2 *µ* g/ml, 50 mM Mes-sodium salt, pH 6.8) was added, and the reaction was allowed to proceed at 37°C for 10 minutes. The cartridge was then washed in the same manner as mentioned above. To the particle-containing cartridge was added 200 *µ* l of a substrate solution containing 200 *µ* g/ml of the luminescent substrate AMPPD (0.1 M DEA-hydrochloride, 1 mM MgCl₂, pH 10.0), and the reaction was allowed to proceed at 37°C for 5 minutes. Thereafter, the luminescence intensity was measured using a luminometer (ALOKA).

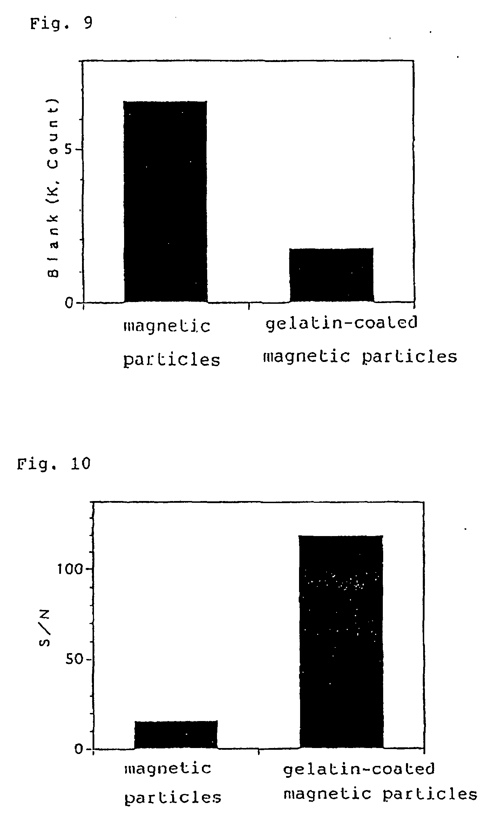

The obtained signal value is shown in Fig. 8, the blank value (noise value) in Fig. 9 and the S/N ratio in Fig. 10.

### INDUSTRIAL APPLICABILITY

The magnetic particles to be used in immunoassay in accordance with this invention are each composed of a core essentially consisting of organic polymer materials, a magnetic material layer coating the core, and gelatin on the surface of said magnetic material layer and, therefore, are substantially free of iron migration and very stable against a prolonged period of storage. They are excellent in magnetic response and hardly undergo self aggregation, so that they can be expected to conform satisfactorily to rapid B/F separation and washing requirements. Therefore, the method of this invention can give low-noise, high-signal assay results.

## Claims

1. A gelatin-coated magnetic particle which comprises a core comprising an organic polymer material, a magnetic material layer coating the surface of said core, and a gelatin layer coating the surface of said magnetic material layer, said particle having a diameter of 1.0 to 10 *µ* m.

2. The gelatin-coated magnetic particle according to claim 1, wherein said core has a diameter of 0.1 to 5 *µ* m.

3. A gelatin-coated magnetic particle according to claim 1 or 2, wherein said an organic polymer material comprises at least one member selected from the group consisting of polystyrene, polymethacrylic esters, polyacrylic esters, styrene-methacrylic ester copolymers, styrene-acrylic ester copolymers, polymerized silanes and organic silanes.

4. An immunoassay method using gelatin-coated magnetic particles comprising a core comprising an organic polymer material, a magnetic material layer coating the surface of said core, and a gelatin layer coating the surface of said magnetic material layer, said particles having a diameter of 1.0 to 10 *µ* m.

5. An immunoassay method according to claim 4, wherein said core has a diameter of 0.1 to 5 *µ* m.

6. An immunoassay method according to claim 4 or 5, wherein said organic polymer material comprises at least one member selected from the group consisting of polystyrene, polymethacrylic esters, polyacrylic esters, styrene-methacrylic ester copolymers, styrene-acrylic ester copolymers, polymerized silanes and organic silanes.

7. An immunoassay method according to claim 4, 5 or 6, wherein labeled antigens or antibodies are used.

## Patentansprüche

1. Mit Gelatine beschichtetes Magnetteilchen, welches einen Kern, der ein organisches Polymermaterial umfaßt, eine Schicht aus magnetischem Material, welche die Oberfläche des Kerns überzieht, und eine Gelatineschicht, welche die Oberfläche der Schicht aus magnetischem Material überzieht, umfaßt, wobei das Teilchen einen Durchmesser von 1,0 bis 10 *µ*m aufweist.

2. Mit Gelatine beschichtetes Magnetteilchen nach Anspruch 1, wobei der Kern einen Durchmesser von 0,1 bis 5 *µ*m aufweist.

3. Mit Gelatine beschichtetes Magnetteilchen nach Anspruch 1 oder 2, wobei das organische Polymermaterial mindestens einen aus Polystyrol, Polymethacrylsäureestern, Polyacrylsäureestem, Styrol-Methacrylsäureester-Copolymeren, Styrol-Acrylsäureester-Copolymeren, polymerisierten Silanen und organischen Silanen ausgewählten Vertreter umfaßt.

4. Immunoassay-Verfahren unter Verwendung von mit Gelatine beschichteten Magnetteilchen, die einen Kern, der ein organisches Polymermaterial umfaßt, eine Schicht aus magnetischem Material, welche die Oberfläche des Kerns überzieht, und eine Gelatineschicht, welche die Oberfläche der Schicht aus magnetischem Material überzieht, umfassen, wobei die Teilchen einen Durchmesser von 1,0 bis 10 *µ*m aufweisen.

5. Immunoassay-Verfahren nach Anspruch 4, bei welchem der Kern einen Durchmesser von 0,1 bis 5 *µ*m aufweist.

6. Immunoassay-Verfahren nach Anspruch 4 oder 5, bei welchem das organische Polymermaterial mindestens einen aus Polystyrol, Polymethacrylsäureestern, Polyacrylsäureestern, Styrol-Methacrylsäureester-Copolymeren, Styrol-Acrylsäureester-Copolymeren, polymerisierten Silanen und organischen Silanen ausgewählten Vertreter umfaßt.

7. Immunoassay-Verfahren nach Anspruch 4, 5 oder 6, bei welchem markierte Antigene oder Antikörper verwendet werden.

## Revendications

1. Particule magnétique enrobée de gélatine comprenant un noyau comprenant une matière polymère organique, une couche de matière magnétique enrobant la surface dudit noyau et une couche de gélatine enrobant la surface de ladite couche de matière magnétique, ladite particule ayant un diamètre de 1,0 à 10 µm.

2. Particule magnétique enrobée de gélatine selon la revendication 1, dans laquelle ledit noyau a un diamètre de 0,1 à 5 µm.

3. Particule magnétique enrobée de gélatine selon la revendication 1 ou 2, dans laquelle ladite matière polymère organique comprend au moins un élément choisi dans le groupe constitué par le polystyrène, les esters polyméthacryliques, les esters polyacryliques, les copolymères styrène - ester méthacrylique, les copolymères styrène - ester acrylique, les silanes polymérisés et les silanes organiques.

4. Méthode de dosage immunologique utilisant des particules magnétiques enrobées de gélatine comprenant un noyau comprenant une matière polymère organique, une couche de matière magnétique enrobant la surface dudit noyau et une couche de gélatine enrobant la surface de ladite couche de matière magnétique, lesdites particules ayant un diamètre de 1,0 à 10 µm.

5. Méthode de dosage immunologique selon la revendication 4, dans laquelle ledit noyau a un diamètre de 0,1 à 5 µm.

6. Méthode de dosage immunologique selon la revendication 4 ou 5, dans laquelle ladite matière polymère organique comprend au moins un élément choisi dans le groupe constitué par le polystyrène, les esters polyméthacryliques, les esters polyacryliques, les copolymères styrène - ester méthacrylique, les copolymères styrène - ester acrylique, les silanes polymérisés et les silanes organiques.

7. Méthode de dosage immunologique selon la revendication 4, 5 ou 6, dans laquelle on utilise des antigènes ou des anticorps marqués.
